# EUROPÄISCHE PATENTANMELDUNG

(11) **EP 2 143 717 A1**
(43) Veröffentlichungstag der Anmeldung: **13.01.2010**
(21) Anmeldenummer: 08450104.8
(22) Anmeldetag: 11.07.2008
(51) Int. Cl.: C07D 251/70, C08G 18/20

(54) **Vinyl-substituerte Melamine**

(71) Anmelder: Johannes Kepler Universität Linz, 4040 Linz (AT)
(72) Erfinder: Schmidt, Harald, o.Univ. Prof. Dr., 4040 Linz (AT); Schwarzinger, Clemens, PD DI Dr., 4600 Wels (AT); Huber, Werner F., DI Dr., 5232 Kirchberg (AT)
(74) Vertreter: Bachinger-Fuchs, Eva-Maria

(57) **Zusammenfassung**

Die Erfindung betrifft Melaminderivate der allgemeinen Formel I 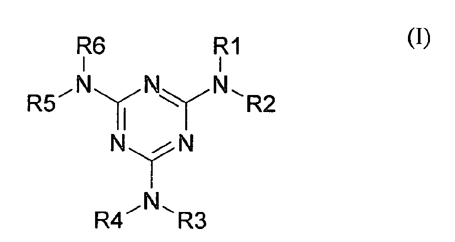 wobei
R1 bis R6 aus der Gruppe, bestehend aus Vinyl, H, (C₁-C₆)-Alkyl, Methylol, CH₂OR7, wobei R7 (C₁-C₄)-Alkyl ist, und Allyl, ausgewählt ist oder zwei an denselben Stickstoff gebundenen Reste gemeinsam mit N einen Phthalimid- oder Succinimidrest bilden, mit der Maßgabe, dass mindestens eines von R1-R6 Vinyl ist.

## Beschreibung

Die vorliegende Erfindung betrifft vinylsubstituierte Melaminderivate der allgemeinen Formel I, daraus erhältliche Polymere sowie ein Verfahren zur Herstellung der Melaminderivate.

In der Literatur sind zahlreiche Melaminderivate beschrieben. Mit Vinyl substituierte Melaminderivate sind jedoch nicht bekannt, da im Unterschied zu vielen anderen Substituenten eine N-Vinyl-Substitution von Melamin oder Melaminderivaten bisher noch nicht gelungen ist.

Mit einer oder mehreren Vinylgruppen substituierte Melamine oder Melaminderivate würden neben einer Kondensationsreaktion an den NH-Bindungen, zum Beispiel mit Formaldehyd, eine weitere polymere Aufbaureaktion mit der Vinylgruppe gestatten oder könnten als neue Vinylmonomere zu Polyvinylmelaminen polymerisiert werden. Außerdem könnten vinylsubstituierten Melaminderivate mit bekannten Vinylmonomeren, wie z.B. Ethylen, Propylen, Vinylchlorid, Styrol, Vinylacetat u. a., copolymerisiert werden.

Derartige Vinylmelamine wären darüber hinaus für auch für neuartige Agrochemikalien und pharmazeutische Produkte von Interesse.

Die Erfindung stellt sich daher die Aufgabe, mit Vinyl substituierte Melaminderivate bereitzustellen, die insbesondere als Ausgangspunkt für die Herstellung neuartiger Polymere eingesetzt werden können. Eine weitere Aufgabe der Erfindung besteht in der Bereitstellung eines Verfahrens zur Herstellung solcher vinylsubstituierten Melaminderivate sowie von Polymeren, die aus diesen Melaminderivat-Monomeren erhältlich sind.

Die Aufgabe wird erfindungsgemäß durch Melaminderivate der allgemeinen Formel I 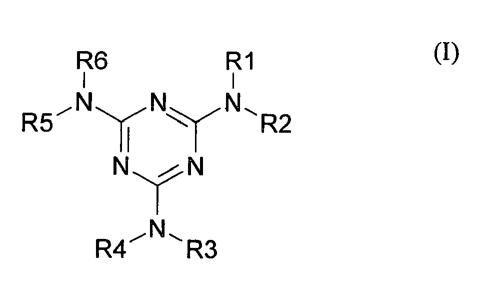 gelöst, wobei
R1 bis R6 aus der Gruppe, bestehend aus Vinyl, H, (C₁-C₆)-Alkyl, Methylol, CH₂OR7, wobei R7 (C₁-C₄)-Alkyl ist, und Allyl, ausgewählt ist oder zwei an denselben Stickstoff gebundenen Reste gemeinsam mit N einen Phthalimid- oder Succinimidrest bilden, mit der Maßgabe, dass mindestens eines von R1-R6 Vinyl ist.

Bevorzugte Melaminderivate sind wie folgt gekennzeichnet:
- R1 = Vinyl, R2 bis R6 = H, (C₁-C₆)-Alkyl, Methylol, CH₂OR7 oder Allyl
- R1 = R2 = Vinyl, R3 bis R6 = H, (C₁-C₆)-Alkyl, Methylol, CH₂OR7 oder Allyl
- R1 = R3 = R5 = Vinyl, R2, R4, R6 = H, (C₁-C₆)-Alkyl, Methylol, CH₂OR7 oder Allyl
- R1 = R2 = R3 = Vinyl, R4 bis R6 = H, (C₁-C₆)-Alkyl, Methylol, CH₂OR7 oder Allyl
- R1 = R2 = R3 = R4 = Vinyl, R5, R6 = H, (C₁-C₆)-Alkyl, Methylol, CH₂OR7 oder Allyl
- R1 = R2 = R3 = R4 = R5 = Vinyl, R6 = H, (C₁-C₆)-Alkyl, Methylol, CH₂OR7 oder Allyl

Das erfindungsgemäße Verfahren zur Herstellung der obigen Melaminderivate ist **dadurch gekennzeichnet, dass** eine Verbindung der Formel II 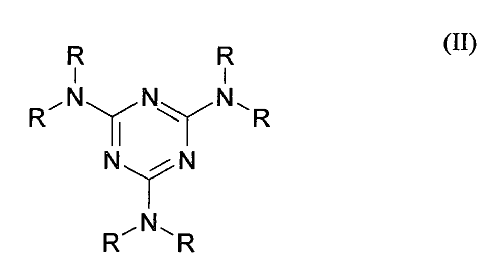 wobei jedes R unabhängig aus der Gruppe, bestehend aus H, (C₁-C₆)-Alkyl, Allyl, Methylol und CH₂OR7, wobei R7 (C₁-C₄)-Alkyl ist, ausgewählt ist oder zwei an denselben Stickstoff gebundene Reste R mit N einen Phthalimid- oder Succinimidrest bilden, mit der Maßgabe, dass mindestens ein R Wasserstoff ist, in Gegenwart eines Vinylierungskatalysators mit Acetylen umgesetzt wird.

Gemäß einer bevorzugten Ausführungsform ist der Vinylisierungskatalysator aus der Gruppe, bestehend aus einer basischen metallorganischen Verbindung, vorzugsweise Natriummethylat oder Kalium-t-butylat, einem Quecksilbersalz, z.B. Quecksilbersulfat, einem Zinksalz, z.B. Zinkacetat, und einem Kupfersalz, z.B. Kupfersulfat, ausgewählt.

Bei Quecksilber- und Kupferverbindungen ist der Vinylierungskatalysator zweckmäßigerweise auf einem Trägermaterial, insbesondere Aktivkohle, Silikat oder Aluminiumoxid, aufgebracht.

Die Reaktion kann je nach Wahl des Lösungsmittels drucklos oder unter Druck durchgeführt werden. Vorzugsweise wird die Vinylierungsreaktion jedoch bei einem Druck von 0,1-2,0 MPa und einer Temperatur von 80°C-190°C durchgeführt. Die Reaktionszeit beträgt bevorzugt 1 bis 12 Stunden.

Die Vinylierung der Melamine mit Acetylen wird vorteilhaft in einem Lösungsmittel, wie Toluol, Tetrahydrofuran, Dimethylsulfoxid, Dioxan oder Mischungen davon, durchgeführt.

Die Darstellung von Methylol-substituierten Vinylmelaminen kann auch nach der Vinylierung durch Umsetzen der Melamine mit Formaldehyd durchgeführt werden. Aus diesen können dann auch durch Reaktion der Methylolgruppe mit den entsprechenden Alkoholen CH₂OR7-substituierte Vinylmelamine hergestellt werden, die z.B. im Bereich der Lackanwendungen von Melaminharzen üblich sind.

Ein weiterer Aspekt der Erfindung betrifft Polymere, welche durch Polymerisation, Copolymerisation oder Polykondensation erhältlich sind, wobei als Monomer ein Melaminderivat der allgemeinen Formel I eingesetzt wird.

Das erfindungsgemäße Melaminderivat wird dabei als Monomer oder als Copolymer mit anderen Vinylmonomeren beispielsweise für die Herstellung von Kunststoffen, Plastifizierungsmitteln oder Lackbindemitteln verwendet. Gemäß einer anderen Ausführungsform wird es als Monomer für Kondesationsreaktionen mit Carbonylverbindungen, insbesondere mit Formaldehyd, für Laminatbeschichtungen, Lackbindemittel und Verleimungsmittel für Holzwerkstoffe eingesetzt. Auch seine Anwendung als Baustein für neue Agrochemikalien und pharmazeutische Produkte wird von der vorliegenden Erfindung umfasst.

Gegenstand der Erfindung sind weiters Verbundstoffe, insbesondere "Wood Polymer Composites" und Glasfaserverbundstoffe, welche ein wie oben definertes Polymer umfassen.

Die Erfindung wird nachfolgend anhand von Beispielen näher erläutert.

### Beispiel 1

In einem 1-Liter-Rührreaktor werden 19,6 g Pentamethylmelamin in 400 ml Dimethylsulfoxid aufgelöst und auf 100°C erwärmt. Nach Zugabe von 0,5 g Kalium-tert.-butylat werden innerhalb von 1 Stunde 2,5 l gasförmiges Acetylen eingeleitet. Danach wird abgekühlt und das Dimethylsulfoxid unter Vakuum abdestilliert. Der verbleibende Rückstand wird in Wasser aufgenommen, 2 mal mit 50 ml Toluol extrahiert und danach der Toluolextrakt eingeengt. Die HPLC-MS Analyse führte zu folgendem Ergebnis:
0,3 % Pentamethylmelamin
98,6 % N-Vinyl-pentamethylmelamin
1,1% Rückstand

Durch Umkristallisieren aus Methanol konnte N-Vinyl-pentamethylmelamin in reiner Form isoliert werden:
Schmelzpunkt: 102°C
H-NMR-Signale: 7,77 - 7,90 ppm (dd, J₁ = 9,22 Hz, J₂ =16,02 Hz, 1H) 4,32 ppm (d, J₂ = 16,02 Hz, 1H) 4,18 ppm (d, J₁ = 9,22 Hz, 1H) 3,19 ppm (s, 3H) 3,06 ppm (s, 12H)
MS: 222 Da
¹³C-NMR, HSQC und HMC bestätigen obige Struktur.

### Beispiel 2

In einem 1-Liter-Rührreaktor werden 18,2 g N,N',N",N"-Tetramethylmelamin in 600 ml Dimethylsulfoxid aufgelöst und auf 130°C erwärmt. Nach Zugabe von 0,3 g Kalium-tert.-butylat werden innerhalb von 2 Stunden 4,5 l gasförmiges Acetylen eingeleitet. Danach wird abgekühlt und das Dimethylsufoxid unter Vakuum abdestilliert. Der verbleibende Rückstand wird gemäß Beispiel 1 aufgearbeitet. Die HPLC-MS Analyse führte zu folgendem Ergebnis:
0,8 % N,N',N",N"-Tetramethylmelamin
27,2% N-Vinyl-N,N',N",N"-tetramethylmelamin (MH⁺=209)
68,7% N,N'-Divinyl-N,N',N",N"-tetramethylmelamin (MH⁺=235)
3,3% Rückstand

Durch mehrfaches Umkristallisieren aus Methanol konnte N,N'-Divinyl-N,N',N",N"-tetramethylmelamin in reiner Form isoliert werden.
Schmelzpunkt: 51°C
HNMR-Signale: 7,75 - 7,82 ppm (dd, J₁ = 9,21 Hz, J₂ =15,92 Hz, 2H) 4,41 ppm (d, J₂ = 15,92 Hz, 2H) 4,27 ppm (d, J₁ = 9,21, Hz 2H) 3,22 ppm (s, 6H) 3,09 ppm (s, 6H)
MS: 234 Da
¹³C-NMR, HSQC und HMC bestätigen obige Struktur

Eine vollständige Isolierung und Auftrennung der einzelnen vinylsubstituierten Melaminderivate kann durch chromatographische Verfahren erreicht werden.

### Analytik des umkristallisierten Produktes:

### Thermo QTrap GC/MS System:

- Säule:: Restek RTX-35 MS (30 m)
- GC Programm:: 50°C (4 min) auf 310°C (5 min) mit 10°C min⁻¹
- Trägergas:: Helium, 2,5 ml min⁻¹
0,55% N,N',N",N"-Tetramethylmelamin (t_{R} = 18,38 min)
0,87% N-Vinyl-N,N',N",N"-tetramethylmelamin (t_{R} = 19,46 min)
98,58% N,N'-Divinyl-N,N',N",N"-tetramethylmelamin (t_{R} = 20,2 min)

### Beispiel 3

In einem 1-Liter-Rührautoklav werden 16,8 g N,N',N"-Trimethylmelamin, 800 ml Toluol und 0,8 g Natriummethylat vorgelegt und unter Kühlung 7,5 g Acetylen einkondensiert. Danach wird auf 165 °C erwärmt und diese Temperatur 3 Stunden gehalten. Nach der Entspannung des Autoklaven wird der Rückstand gemäß Beispiel 1 aufgearbeitet. Die Analyse ergab folgende Zusammensetzung:
2,7 % N,N',N"-Trimethylmelamin
20,3 % N-Vinyl-N,N'N"-trimethylmelamin (MH⁺=195)
37,1 % N,N'-Divinyl-N,N'N"-trimethylmelamin (MH⁺=221)
35,4 % N,N',N"-Trivinyl-N,N',N"-trimethylmelamin (NH⁺=247)
4,5 % Rückstand

Eine vollständige Isolierung und Auftrennung der einzelnen vinylsubstituierten Melaminderivate kann durch chromatographische Verfahren erreicht werden.

### Beispiel 4

In einem 1-Liter-Rührautoklav werden 15,4 g N,N',-Dimethylmelamin, 400 ml Toluol, 200 ml Dimethylsulfoxid und 0,8 g Natriummethylat vorgelegt und unter Kühlung 12,5 g Acetylen einkondensiert. Danach wird auf 155 °C erwärmt und diese Temperatur 5 Stunden gehalten. Nach der Entspannung des Autoklaven wird der Rückstand gemäß Beispiel 1 aufgearbeitet. Die Analyse ergab folgende Zusammensetzung:
8,7 % N,N'-Dimethylmelamin
17,2 % N-Vinyl-N,N'-dimethylmelamin (MH⁺=181)
26,1 % N,N'-divinyl-N,N'-dimethylmelamin (MH⁺=207)
35,4 % N,N',N"-Trivinyl-N,N'-dimethylmelamin (MH⁺=233)
11,9 % N,N',N",N"-Tetravinyl-N,N'-dimethylmelamin (MH⁺=259)
1,7 % Rückstand

Eine vollständige Isolierung und Auftrennung der einzelnen vinylsubstituierten Melaminderivate kann durch chromatographische Verfahren erreicht werden.

### Beispiel 5

In einem 1-Liter-Rührautoklav werden 15,4 g N,N',N"-Triallylmelamin, 800 ml Toluol und 0,6 g Natriummethylat vorgelegt und unter Kühlung 7,5 g Acetylen einkondensiert. Danach wird auf 140°C erwärmt und diese Temperatur 2,5 Stunden gehalten. Nach der Entspannung des Autoklaven wird der Rückstand gemäß Beispiel 1 aufgearbeitet. Die Analyse ergab folgende Zusammensetzung:
8,3% N,N'N"-Triallylmelamin
11,7 % N-Vinyl- N,N',N"-triallylmelamin (MH⁺=273)
37,2 % N,N'-Divinyl-N,N'-N"-triallylmelamin (ME⁺=299)
30,8 % N,N'N"-Trivinyl-N,N',N"-triallylmelamin (MH⁺=325)
2,0% Rückstand

Eine vollständige Isolierung und Auftrennung der einzelnen vinylsubstituierten Melaminderivate kann durch chromatographische Verfahren erreicht werden.

### Beispiel 6

In einem 1-Liter-Rührautoklav werden 21,0 g N,N',N"-Triethylmelamin, 400 ml Toluol, 200 ml Tetrahydrofuran und 0,6 g Natriummethylat vorgelegt und unter Kühlung 8,5 g Acetylen einkondensiert. Danach wird auf 180°C erwärmt und diese Temperatur 2 Stunden gehalten. Nach der Entspannung des Autoklaven wird der Rückstand gemäß Beispiel 1 aufgearbeitet. Die Analyse ergab folgende Zusammensetzung:
1,9 % N,N',N"-Triethylmelamin
21,5 % N-Vinyl-N,N'N"-triethylmelamin (MH⁺=237)
34,0 % N,N'-Divinyl-N,N'N"-triethylmelamin (MH⁺=263)
37,4 % N,N',N"-Trivinyl-N,N',N"-triethylmelamin (MH⁺=289)
5,2 % Rückstand

Eine vollständige Isolierung und Auftrennung der einzelnen vinylsubstituierten Melaminderivate kann durch chromatographische Verfahren erreicht werden.

### Beispiel 7

In einem 2-Liter-Rührreaktor werden 12,8 g Phthalimidomelamin in 1000 ml Dimethylsulfoxid aufgelöst und auf 150°C erwärmt. Nach Zugabe von 0,8 g Quecksilber-(II)-sulfat werden innerhalb von 4 Stunden 5,5 Liter gasförmiges Acetylen eingeleitet. Danach wird abgekühlt und das Dimethylsulfoxid unter Vakuum abdestilliert. Der verbleibende Rückstand wird gemäß Beispiel 1 aufgearbeitet. Die HPLC-MS -Analyse führte zu folgendem Ergebnis:
22,8 % Phthalimidomelamin
12,1 % N-Vinyl-phthalimidomelamin (MH⁺=283)
1,2 % N,N-Divinyl-phthalimidomelamin (MH⁺=309)
18,3 % N,N'-Divinyl-phthalimidomelamin (MH⁺=309)
3,2 % N,N,N'-Trivinyl-phthalimidomelamin (MH⁺=335)
21,0 % N,N,N',N'-Tetravinyl-phthalimidomelamin (MH⁺=361)
21,4% Rückstand

Eine vollständige Isolierung und Auftrennung der einzelnen vinylsubstituierten Melaminderivate kann durch chromatographische Verfahren erreicht werden.

### Beispiel 8

In einem 2-Liter-Rührautoklav werden 12,6 g Melamin in 200 ml Toluol, 800 ml Dimethylsulfoxid und 0,6 g Kupfer-(I)-chlorid vorgelegt und unter Kühlung 10,5 g Acetylen einkondensiert. Danach wird auf 180°C erwärmt und diese Temperatur 4 Stunden gehalten. Nach der Entspannung des Autoklaven wird der Rückstand gemäß Beispiel 1 aufgearbeitet. Die Analyse ergab folgende Zusammensetzung:
25,3 % Melamin
12,1 % N-Vinylmelamin (MH⁺=153)
18,4 % N,N'-Divinylmelamin (MH⁺=179)
33,7 % N,N',N"-Trivinylmelamin (MH⁺=205)
12,5% Rückstand

Eine vollständige Isolierung und Auftrennung der einzelnen vinylsubstituierten Melaminderivate kann durch chromatographische Verfahren erreicht werden.

### Beispiel 9

In einem 2-Liter-Rührautoklav werden 12,6 g Melamin in 1000 ml Dimethylsulfoxid und 2,0 g Katalysator (15 % Quecksilber-(II)-sulfat auf Aktivkohle) vorgelegt und unter Kühlung 10,5 g Acetylen einkondensiert. Danach wird auf 190°C erwärmt und diese Temperatur 3,5 Stunden gehalten. Nach der Entspannung des Autoklaven wird der Katalysator abfiltriert und der flüssige Anteil gemäß Beispiel 1 aufgearbeitet. Die Analyse ergab folgende Zusammensetzung:
20,1 % Melamin
14,1 % N-Vinylmelamin (MH⁺=153)
21,0 % N,N'-Divinylmelamin (MH⁺=179)
32,7 % N,N',N"-Trivinylmelamin (MH⁺=205)
12,1 % Rückstand

Eine vollständige Isolierung und Auftrennung der einzelnen vinylsubstituierten Melaminderivate kann durch chromatographische Verfahren erreicht werden.

### Beispiel 10

In einem 1-Liter-Rührautoklav werden 16,8 g N,N',N"-Trimethylmelamin, 1000 ml Dioxan und 0,8 g Zinkacetat vorgelegt und unter Kühlung werden 7,5 g Acetylen einkondensiert. Danach wird auf 160°C erwärmt und diese Temperatur 2,5 Stunden gehalten. Nach der Entspannung des Autoklaven wird der Rückstand gemäß Beispiel 1 aufgearbeitet. Die Analyse ergab folgende Zusammensetzung:
2,1 % N,N',N"-Trimethylmelamin
22,4 % N-Vinyl-N,N'N"-trimethylmelamin (MH⁺=195)
34,1 % N,N'-Divinyl-N,N'N"-trimethylmelamin (MH⁺=221)
31,7 % N,N',N"-Trivinyl-N,N',N"-trimethylmelamin (MH⁺=247)
9,7 % Rückstand

Eine vollständige Isolierung und Auftrennung der einzelnen vinylsubstituierten Melaminderivate kann durch chromatographische Verfahren erreicht werden.

### Beispiel 11

In einem 100-ml-Dreihalskolben mit Rückflusskühler und pH-Elektrode werden 35 ml 33%ige Formalinlösung vorgelegt und mit Natronlauge wird ein pH von 8,4 eingestellt. Die Lösung wird auf 80°C erwärmt und unter Rühren werden 20 g einer Mischung aus 34 % N-Vinyl-N,N'N"-trimethylmelamin, 63 % N,N'-Divinyl-N,N'N"-trimethylmelamin und 3 % N,N',N"-Trivinyl-N,N',N"-trimethylmelamin zugegeben. Nach etwa 10 Minuten ist die Lösung noch leicht trüb. Nach einer weiteren Reaktionszeit von 10 Minuten wird die Lösung auf Raumtemperatur abgekühlt. Der Methylolgruppengehalt der Lösung wird nach der Cyanidmethode und mit Hilfe der ¹H-NMR bestimmt. Das Gemisch ist vollständig methyloliert, freie NH-Gruppen können nicht nachgewiesen werden. Identifizierung der einzelnen Bestandteile mittels ESI-MS:
N-Vinyl-N,N'N"-trimethyldimethylolmelamin (MH⁺=255)
N,N'-Divinyl-N,N'N"-trimethylmethylolmelamin (MH⁺=251)

Eine vollständige Isolierung und Auftrennung der einzelnen vinylsubstituierten Melaminderivate kann durch chromatographische Verfahren erreicht werden.

### Beispiel 12

0,1 g Tetramethyldivinylmelamin werden mit 5% (w/w) Dibenzoylperoxid gemischt und in einen Aluminiumtiegel überführt. Die Mischung wird in einer DSC mit einer Heizrate von 10°C min⁻¹ polymerisiert. Es wird ein Schmelzpeak bei 50-60°C (endotherm) und ein Polymerisationspeak bei 107-150°C (exotherm, Maximum bei 132,5°C) detektiert.

### Beispiel 13

20 g Alkylvinylmelamine aus Beispiel 3 werden mit 5% (w/w) Dibenzoylperoxid und 40 g Holzspänen des Typs BK70/90 (Nadelholz) gemischt und in eine Aluminiumform gefüllt. In einer Tischpresse wird die Mischung bei 160°C und 50 bar für 10 Minuten gepresst und danach noch unter Druck abgekühlt. Nach dem Erkalten lässt sich eine homogene, feste Spanplatte aus der Form lösen.

### Beispiel 14

1 g Pentamethylvinylmelamin wird mit 4 g Styrol und 5% (w/w) Dibenzoylperoxid gemischt und in einen Glaskolben mit Magnetrührer überführt. Die Mischung wird mittels Ölbad auf 160°C erwärmt. Es bildet sich eine homogene, immer viskoser werdende Masse. Nach 2 Stunden wird die Mischung auf Raumtemperatur abgekühlt und bildet eine klare, harte Masse.

## Patentansprüche

1. Melaminderivat der allgemeinen Formel I 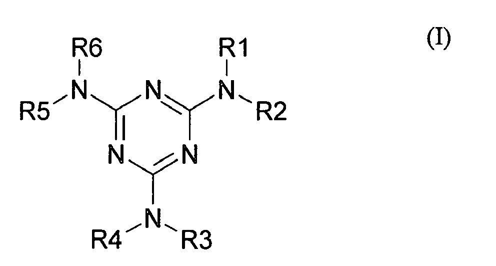 wobei
R1 bis R6 aus der Gruppe, bestehend aus Vinyl, H, (C₁-C₆)-Alkyl, Methylol, CH₂OR7, wobei R7 (C₁-C₄)-Alkyl ist, und Allyl, ausgewählt ist oder zwei an denselben Stickstoff gebundenen Reste gemeinsam mit N einen Phthalimid- oder Succinimidrest bilden,
mit der Maßgabe, dass mindestens eines von R1-R6 Vinyl ist.

2. Melaminderivat nach Anspruch 1, **dadurch gekennzeichnet, dass** R1 = Vinyl, R2 bis R6 = H, (C₁-C₆)-Alkyl, Methylol, CH₂OR7 oder Allyl.

3. Melaminderivat nach Anspruch 1, **dadurch gekennzeichnet, dass** R1 = R2 = Vinyl, R3 bis R6 = H, (C₁-C₆)-Alkyl, Methylol, CH₂OR7 oder Allyl.

4. Melaminderivat nach Anspruch 1, **dadurch gekennzeichnet, dass** R1 = R3 = R5 = Vinyl, R2, R4, R6 = H, (C₁-C₆)-Alkyl, Methylol, CH₂OR7 oder Allyl.

5. Melaminderivat nach Anspruch 1, **dadurch gekennzeichnet, dass** R1 = R2 = R3 = Vinyl, R4 bis R6 = H, (C₁-C₆)-Alkyl, Methylol, CH₂OR7 oder Allyl.

6. Melaminderivat nach Anspruch 1, **dadurch gekennzeichnet, dass** R1 = R2 = R3 = R4 = Vinyl, R5, R6 = H, (C₁-C₆)-Alkyl, Methylol, CH₂OR7 oder Allyl.

7. Melaminderivat nach Anspruch 1, **dadurch gekennzeichnet, dass** R1 = R2 = R3 = R4 = R5 = Vinyl, R6 = H, (C₁-C₆)-Alkyl, Methylol, CH₂OR7 oder Allyl.

8. Verfahren zur Herstellung eines Melaminderivats der allgemeinen Formel I, **dadurch gekennzeichnet, dass** eine Verbindung der Formel II 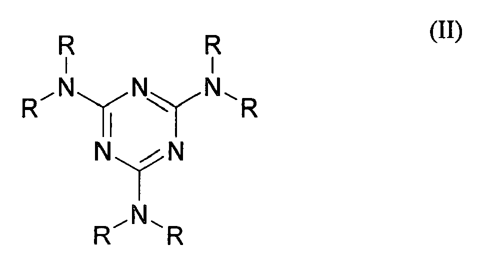 wobei jedes R unabhängig aus der Gruppe, bestehend aus H, (C₁-C₆)-Alkyl, Allyl, Methylol und CH₂OR7, wobei R7 (C₁-C₄)-Alkyl ist, ausgewählt ist oder zwei an denselben Stickstoff gebundene Reste R mit N einen Phthalimid- oder Succinimidrest bilden, mit der Maßgabe, dass mindestens ein R Wasserstoff ist,
in Gegenwart eines Vinylierungskatalysators mit Acetylen umgesetzt wird.

9. Verfahren nach Anspruch 8, **dadurch gekennzeichnet, dass** der Vinylisierungskatalysator aus der Gruppe, bestehend aus einer basischen metallorganischen Verbindung, vorzugsweise Natriummethylat oder Kalium-t-butylat, einem Quecksilbersalz, einem Zinksalz und einem Kupfersalz, ausgewählt ist.

10. Verfahren nach Anspruch 8 oder 9, **dadurch gekennzeichnet, dass** die Vinylierungsreaktion bei einem Druck von 0,1-2,0 MPa und einer Temperatur von 80°C-190°C durchgeführt wird.

11. Polymer, erhältlich durch Polymerisation, Copolymerisation oder Polykondensation, wobei als Monomer ein Melaminderivat der allgemeinen Formel I eingesetzt wird.

12. Verbundstoff, umfassend ein Polymer nach Anspruch 11.
